# FASCICULE DE BREVET EUROPEEN

(11) **EP 1 774 336 B1**
(45) Date de publication et mention de la délivrance du brevet: **20.07.2011**
(21) Numéro de dépôt: 05793374.9
(22) Date de dépôt: 29.07.2005
(51) Int. Cl.: G01N 33/543

(54) **METHODE DE DETERMINATION DU STATUT VACCINAL DE PERSONNES**
VERFAHREN ZUM NACHWEIS DES IMMUNISIERUNGSZUSTANDS EINER PERSON
METHOD FOR DETERMINING THE IMMUNISATION STATUS OF A PERSON

(30) Priorité: 03.08.2004 FR 0408600
(43) Date de publication de la demande: 18.04.2007
(73) Titulaire: INODIAG, 83870 Signes (FR)
(72) Inventeur: ESCARGUEL, Claude, F-83110 Sanary-sur-Mer (FR)
(74) Mandataire: Domange, Maxime
(86) Numéro de dépôt international: PCT/FR2005/001990
(87) Numéro de publication internationale: WO 2006/024761

(56) Documents cités:
- WO-A-00/77518
- WO-A-01/20325
- WO-A-03/046561
- ASARI SEISHI ET AL: "Seroprevalence survey of measles, rubella, varicella, and mumps antibodies in health care workers and evaluation of a vaccination program in a tertiary care hospital in Japan." AMERICAN JOURNAL OF INFECTION CONTROL. MAY 2003, vol. 31, no. 3, mai 2003 (2003-05), pages 157-162, XP002331428 ISSN: 0196-6553 cité dans la demande

## Description

La présente invention concerne une méthode et une trousse de diagnostic pour la détermination sérologique du statut vaccinal de personnes. La vaccination consiste à introduire par une voie d'administration appropriée un ou plusieurs antigènes d'un agent pathogène bactérien, viral ou parasitaire, éventuellement associé à une ou plusieurs substances stimulant la réponse immunitaire contre ce (ces) antigènes (adjuvants vaccinaux).

Quelque soit le mode de vaccination, l'objectif est la mise en place chez la personne vaccinée d'une réponse immunitaire spécifique du pathogène comportant une immunité mémoire, c'est à dire susceptible de répondre en quelques heures à une nouvelle introduction du pathogène protégeant ainsi efficacement la personne contre le pathogène. Une modalité particulière et essentielle de l'immunité conférée par la vaccination est la production d'anticorps spécifiques de l'antigène vaccinal.

La protection conférée par la vaccination n'est pas permanente, et ne reste acquise que pour un laps de temps variant de quelques mois à quelques décennies. En effet, le pathogène contre lequel est réalisée la vaccination, peut présenter des modifications périodiques de sa composition antigénique rendant partiellement ou complètement caduque la protection conférée par la vaccination avec la composition antigénique précédente. Egalement, la prévalence relative de différents variants antigéniques (sérotypes) du pathogène en fonction du temps et du pays considéré, oblige à modifier la composition du vaccin pour y incorporer les antigènes des sérotypes nouvellement prévalents. Ceci est illustré par les recommandations vaccinales spécifiquement élaborées pour les voyageurs qui se rendent dans les pays de forte endémie de ces pathogènes [Mackell SM. Vaccinations for the pediatric traveler. Clin. Infect. Dis. 2003,37 :1508-1515 ; Kirkpatrick B.D & Kemper Alston W. Current immunization for traval. Current Opinion in Infections Diseases 2003,16:369-374]. Par ailleurs, la durée de vie des anticorps protecteurs induits par le vaccin est limitée à quelques années en l'absence de nouvelle stimulation antigénique par contact avec le pathogène, ou par une nouvelle vaccination. De même, la réponse individuelle peut varier selon l'état immunitaire au moment de la vaccination et selon la voie d'administration. Ces trois raisons, modifications antigéniques du pathogène, disparition progressive des anticorps protecteurs spécifiques et hétérogénéité de réponse imposent une re-vaccination des personnes sous forme de rappels vaccinaux.

La vaccination et l'administration d'un rappel vaccinal peuvent être analysées en tenant compte des effets secondaires connus de certains vaccins, d'une analyse coût/bénéfice, et du libre choix de la personne pour les vaccins qui ne sont pas obligatoires dans le cadre des recommandations publiées sous forme d'un calendrier vaccinal qui précise les modalités d'administration du vaccin et des éventuels rappels de celui-ci [anonyme - Calendrier vaccinal 2003, Avis du Conseil Supérieur d'hygiène publique de France. Bulletin Epidémiologique hebdomadaire 2003, 6 :33-36]. En effet, l'attitude de rappel vaccinal systématique de la population est de plus en plus mise en cause du fait des analyses coût-bénéfice et de la pression de l'opinion publique. Une décision de rappel vaccinal adapté individuellement au statut immunitaire de la personne est donc souhaitable. En effet, le coût de la vaccination est un enjeu considérable non seulement pour les pays industrialisés mais surtout pour les pays en développement (PED) [Carabin H & Edmunds WJ. Future savings form measles eradication in industrialized countries. J.Infect. Dis.2003,187 (suppl 1) :529-535].

La détermination dans le sérum de la personne à vacciner de la présence voire du taux d'anticorps protecteurs spécifiques devrait être un élément clé pour décider l'administration du vaccin ou du rappel vaccinal. En effet, si le sérum de la personne contient des anticorps spécifiques du pathogène à une concentration protectrice, il n'y a aucun bénéfice mais au contraire une prise de risque à administrer un rappel vaccinal à cette personne.

La recherche de la présence d'anticorps de classe IgG spécifiques est actuellement réalisée dans les laboratoires pour les virus de la rougeole, des oreillons et de la rubéole. La détermination au laboratoire de la concentration d'anticorps spécifiques n'est réalisée que pour le virus B de l'hépatite : un taux d'anticorps anti HBs > 10 mUl/ml est considéré comme protecteur contre l'infection par le virus B de l'hépatite et ne justifie pas de rappel vaccinal. Pour la détection des anticorps antitétaniques (dirigé contre la toxine tétanique), quelques tests rapides basés sur une technique d'hémagglutination passive (Vacci-test® Pasteur®) ou sur l'immunochromatographie ont été développés pour améliorer la prise en charge des blessés.

Dans les méthodes proposées actuellement, la détection de la présence ou la mesure de la concentration des anticorps spécifiquement dirigés contre les différents antigènes vaccinaux sont réalisées séparément les unes des autres. Il faut donc réaliser plusieurs prélèvements de sérum qui sont analysés séparément, le cas échéant dans plusieurs laboratoires disposant chacun d'une capacité d'analyse d'un seul antigène vaccinal ou bien d'un nombre restreint d'antigènes vaccinaux.

Quelques études mentionnent la détermination d'une partie du statut vaccinal de la personne. Par exemple, une étude de séroprévalence contre les virus des oreillons, de la rougeole, de la rubéole et de la varicelle a été réalisée chez des personnels soignants au Japon dans le cadre d'un programme vaccinal contre ces pathogènes [Asari S. et al. Seroprevalence survey of measles, rubella, varicella and mumps antibodies in health care workers and evaluation of a vaccination program in a tertiary care hospital in Japan; Am. J. Infect. Control. 2003,31 :157-162]. Dans ce travail, un seul prélèvement sanguin a été réalisé, mais les quatre tests sérologiques, bien que réalisés par le même laboratoire, étaient réalisés en utilisant des plaques de micro-titration différentes sur chacune desquelles était fixé un antigène d'un seul agent pathogène et selon des méthodes différentes. Les méthodes ELISA mises en oeuvre ne permettaient pas de déterminer le titre des anticorps spécifiques testés. Les méthodes ELISA mises en oeuvre impliquaient, en outre, compte tenu de la nature du support solide mise en oeuvre, le prélèvement et l'analyse sur des échantillons de volume relativement important de sérum, à savoir un volume nécessaire pour remplir plusieurs puits de microplaques. Ce travail n'a pas permis de déterminer une séroconversion contre les antigènes spécifiques après vaccination chez 20% des personnes vaccinées contre la rougeole et contre la rubéole et chez 50% des personnes vaccinées contre les oreillons, imputable a une sensibilité médiocre des méthodes de détection mises en oeuvre. Les différentes méthodes proposées pour la détermination du statut vaccinal impliquent souvent des délais de réponse pour obtenir les résultats supérieurs à 24 heures et, en général, avec une sensibilité et donc une fiabilité médiocres.

Il n'existe pas actuellement de trousse, ni de test automatisé et reproductible, permettant la détermination du statut vaccinal d'une personne par la détermination du titre d'anticorps spécifiques contre les principaux pathogènes vaccinaux. Ainsi, il n'est pas fourni actuellement de méthode et de kit permettant de déterminer dans un laps de temps inférieur à 24 heures sur un seul prélèvement de sérum, le statut vaccinal d'une personne, c'est-à-dire la détection ou la détermination des concentrations d'anticorps de classe IgG spécifiques en liaison avec l'ensemble des vaccins actuellement disponibles.

Le but de la présente invention est de fournir une technique de détermination du statut vaccinal d'une personne qui soit simple, rapide et peu coûteuse, utilisable notamment par tout laboratoire pour connaître simultanément par analyse d'un même échantillon de sérum à tester, la concentration d'anticorps contre une pluralité de vaccins actuellement disponibles, et ce sur un volume d'échantillons relativement réduit et donc compatible pour des prélèvements chez l'enfant, y compris le nourrisson.

Plus particulièrement encore, la technique de diagnostic doit pouvoir être automatisable et reproductible de façon fiable, aussi bien dans sa mise en oeuvre que dans la préparation des supports solides mis en oeuvre.

Pour ce faire, la présente invention fournit une méthode de détermination sérologique du statut vaccinal d'un individu par détection et quantification des anticorps sériques du type IgG spécifiques d'antigènes vaccinaux d'une pluralité d'agents pathogènes du type bactéries, virus, champignons ou parasites, caractérisée en ce que l'on réalise la détection et la quantification, d'un complexe de réactions immunologiques entre chaque dit antigène vaccinal et respectivement chaque dit anticorps spécifique de type IgG dudit antigène vaccinal, éventuellement présents dans un échantillon de sérum humain à tester, en réalisant les étapes de :
1- mise en contact d'un seul et même dit échantillon de sérum à tester avec :
   - un même support solide sur lequel est fixé une pluralité de dits antigènes vaccinaux correspondant à une pluralité d'agents pathogènes différents, de préférence au moins 3, de préférence encore au moins 4 antigènes vaccinaux d'agents pathogènes différents, dans des zones différentes du support,
   - en présence d'au moins une substance de détection réagissant par complexation avec lesdits anticorps spécifiques de type IgG et ne réagissant pas avec lesdits antigènes vaccinaux, et
2- on détermine la concentration en dits anticorps spécifiques de type IgG par quantification des complexes résultant de la réaction d'au moins une dite substance de détection avec desdits anticorps spécifiques de type IgG complexés auxdits antigènes vaccinaux fixés sur ledit support solide.

Après lavage du support solide, on vérifie la présence de dits anticorps spécifiques dans l'échantillon en détectant le signal de l'élément de marquage de ladite substance de détection au niveau du site de fixation de l'antigène vaccinal correspondant audit anticorps spécifique sur le support solide, dans la mesure où ladite substance de détection réagit spécifiquement avec ledit anticorps spécifique et ne réagit pas avec ledit antigène vaccinal.

Typiquement, ladite substance de détection est un anticorps secondaire d'origine animale.

Dans un mode préféré de réalisation, ladite substance de détection comprend un marquage fluorescent.

L'expression "marquage fluorescent" signifie que la substance de détection, notamment l'anticorps secondaire de détection a été rendu fluorescent par couplage ou complexation avec une substance fluorescente appropriée telle que l'iso(thio)cyanate de fluorescéine, à savoir une substance émettant un rayonnement détectable après son illumination, chaque dite substance fluorescente étant caractérisée par la longueur d'onde à laquelle elle doit être illuminée (longueur d'onde d'excitation) et la longueur d'onde du rayonnement qu'elle émet (longueur d'onde d'émission).

Comme substance fluorescente de marquage, on peut citer notamment la fluorescéine, la coumarine, la cyanine et les analogues et dérivés de ces substances connues de l'homme de l'art.

La quantification est réalisée par comparaison du signal fluorescent émis par le complexe de réaction [antigène-anticorps spécifique-substance de détection] du sérum testé avec une courbe de référence obtenue par calibration à l'aide de sérums témoins contenant une concentration connue d'anticorps à détecter.

Lorsque l'on utilise une substance fluorescente, la fluorescence associée à l'échantillon testé est lue directement sur un appareil approprié capable de détecter le rayonnement à la longueur d'onde d'émission et de le quantifier.

De tels appareils sont connus de l'homme de l'art.

Le marquage fluorescent est particulièrement avantageux dans le cas de détermination du statut vaccinal dans la mesure où il est essentiel d'obtenir une quantification précise et fiable à la concentration en dits anticorps spécifiques de type IgG, ce qui peut être obtenu grâce aux signaux fluorescents dont l'intensité est directement proportionnelle à la quantité de molécules fluorescentes émettant le signal

On entend par "antigène vaccinal", un antigène susceptible de stimuler une réponse immunitaire du patient induisant la production d'anticorps sériques protecteurs ,c'est à dire un anticorps se liant à l'agent pathogène de telle façon que l'organisme se trouve ainsi protégé contre les effets pathogènes dudit agent.

Avantageusement, on détermine si la concentration de dit anticorps spécifiques atteint un seuil donné à partir duquel ledit anticorps spécifique de type IgG a une action protectrice protégeant contre la maladie déterminée par le pathogène.

Dans certains cas, il est en effet possible de déterminer une concentration d'anticorps spécifiques conférant une protection dans près de 100% des personnes vaccinées, la détermination de cette concentration (ou seuil) est faite par des études séro-épidémiologiques sur de vastes populations.

Dans un mode préféré de réalisation, lesdits antigènes vaccinaux comprennent au moins 3, de préférence de 3 à 12 antigènes vaccinaux d'agents pathogènes choisis parmi les virus des oreillons, de la rubéole, de la rougeole, de la varicelle, de la poliomyélite, de la fièvre jaune, de l'encéphalite à tique, de l'hépatite A, de l'hépatite B, et les bactéries de la coqueluche *Bordetella pertussis,* du tétanos et de la diphtérie.

De préférence, dans la méthode selon l'invention, on utilise une même dite substance de détection pour la détection des différents anticorps spécifiques des différents antigènes vaccinaux.

De préférence encore, et plus particulièrement, on met en oeuvre une dite substance de détection qui est une immunoglobuline anti-IgG, de préférence une immunoglobuline de chèvre ou de poulet.

La présente invention concerne donc plus particulièrement une méthode de diagnostic du statut vaccinal des maladies infectieuses humaines, mais indirecte, qui repose sur la recherche et la quantification dans le sérum du patient d'anticorps spécifiques d'un agent vaccinal de l'agent microbien infectieux, à savoir une bactérie, un virus, un parasite ou un champignon responsable d'une pathologie (désignés ci-dessous "microbe").

Cette méthode selon l'invention est plus particulièrement avantageuse lorsque qu'au moins un dit antigène vaccinal est un antigène microbien corpusculaire constitué de microbe entier inactivé ou fraction de microbe, notamment un antigène vaccinal sous forme de suspension virale de virus entiers vivants désactivés.

Les inventeurs ont en effet développé une technologie par laquelle ce type d'antigène particulaire ou corpusculaire peut être fixé sur le support solide par simple dépose et adsorption physique ou liaison physico-chimique avec le support par une méthode de préparation de support solide selon l'invention comme il sera explicité ci-après. Ces antigènes particulaires ou corpusculaires ont l'avantage d'être particulièrement bien visibles après dépôt sur le support solide et, notamment, lors de la lecture de détection de réactions immunologiques éventuelles.

On utilise donc avantageusement, comme antigènes vaccinaux, des antigènes constitués de microbes entiers inactivés ou de fractions ou fragments de microbes comprenant un ou plusieurs antigènes. Il est en effet possible de fragmenter mécaniquement le microbe par agitation mécanique ou par sonication par exemple ou bien de fragmenter le microbe par un procédé enzymatique afin d'en obtenir une fraction conservant les antigènes qui supportent la réaction sérologique, objet de la présente invention. Les fractions ainsi obtenues sont séparées ou encore purifiées des autres constituant du microbe et de son milieu de culture par un procédé approprié, par exemple par centrifugation ou par filtration. On parle alors de fraction antigénique ou d'antigène purifié. Le microbe entier ou une quelconque fraction du microbe est appelé ci-après "antigène particulaire ou corpusculaire" en ce que le microbe entier ou une de ses fractions ne peut pas être mis en solution par dissolution, mais uniquement en suspension dans un fluide approprié.

Les microbes ou leur fraction ainsi déposés sont remarquablement nettement visibles en tant que particules individualisées par observation microscopique à l'aide d'un microscope optique ou d'un microscope électronique par exemple.

Techniquement, la sérologie microbienne consiste à détecter dans le sérum du patient une réaction antigène - anticorps dans laquelle l'antigène est représenté par tout ou fraction de l'agent microbien infectieux à détecter et l'anticorps est représenté par les immunoglobulines humaines ou animales spécifiques dudit agent microbien infectieux présentes dans le sérum du patient. Elle peut être quantifiée en testant successivement une série de dilutions croissantes de raison 2 ou de raison 10 du sérum du patient à partir d'une première dilution au 1/16 ou bien au 1/50 du sérum du patient.

Classiquement, une méthode de diagnostic sérologique indirecte comporte plus particulièrement le dépôt de l'antigène sur un support solide tel que des microbilles de latex, notamment dans la technique de détection par agglutination dans laquelle des microbilles de latex sont recouvertes par l'antigène microbien à tester, et sont agglutinées les unes aux autres par le sérum de patient présentant des anticorps spécifiques, cette agglutination étant visible à l'oeil nu.

Toutefois, la technique de détection par test d'agglutination est à la fois peu pratique à mettre en oeuvre et peu sensible. Elle implique, en outre, l'utilisation de quantités importantes de réactifs et du sérum du patient et, enfin, la lecture des résultats n'est pas automatisable.

Selon la méthode de l'invention, on dépose donc, le cas échéant, l'antigène sur un support solide du type lame de verre, ou microplaque de titration pour mettre en oeuvre des techniques de détection par immunodétection, de préférence immunofluorescence. Ces techniques de détection sont bien connues de l'homme de l'art, et comprennent les étapes successives de :
1. décomplémentation du sérum par chauffage à 56°C pendant 30 minutes,
2. mise en contact de l'antigène correspondant à l'agent microbien infectieux fixé sur support solide, avec le sérum du patient puis incubation sous des conditions de temps, de température, d'hygrométrie, d'agitation mécanique et de force ionique du milieu permettant la réaction antigène - anticorps,
3. lavage précautionneux et extensif permettant d'éliminer le sérum du patient non-fixé au support solide, en excès ,
4. application d'un anticorps secondaire de détection qui est une immunoglobuline animale dirigée contre les immunoglobulines de l'espèce du patient considéré, par exemple dans le cas d'un patient humain une immunoglobuline de chèvre anti-immunoglobuline humaine conjuguée à une substance fluorochrome, généralement de l'iso-thio-cyanate de fluoresceine et incubation sous des conditions de temps, de température, d'hygrométrie, d'agitation mécanique et de force ionique du milieu permettant la réaction antigène - anticorps,
5. lavage précautionneux et extensif permettant d'éliminer l'immunoglobuline marquée, non fixée, en excès,
6. détection d'une réaction par lecture, à l'aide d'un appareillage approprié en fonction du marqueur tel qu'un microscope à fluorescence ou un lecteur de puces biologiques (microarray en anglais) pour la technique d'immunofluorescence indirecte, connue de l'homme de l'art.

Avantageusement, on utilise une dite substance de détection qui est un anticorps secondaire de détection, une immunoglobuline animale dirigée contre les immunoglobulines de l'espèce du patient considéré, par exemple dans le cas d'un patient humain une immunoglobuline de chèvre anti-immunoglobuline humaine anti IgG humaines qui réagissent avec les immunoglobulines spécifiques de classe G complexées au dit antigène vaccinal.

Aucun test sérologique commercialisé actuellement n'inclut systématiquement le contrôle de l'introduction et de la valeur réactive (ou réactivité immunologique) des anticorps secondaires de détection anti IgG mis en oeuvre, et un autre but original de la présente invention est donc de fournir un test comprenant ce contrôle de réactivité.

De préférence, dans la méthode selon l'invention, on réalise au préalable le contrôle de la présence et la réactivité de la dite première substance de détection en réalisant les étapes de :
- mise en contact dudit échantillon à tester avec un même dit support solide, sur lequel a été fixé en outre un premier antigène de contrôle qui est une immunoglobuline non spécifique de classe G, en présence une solution contenant une dite première substance de détection, et
- vérification si ladite substance de détection a réagi avec ledit premier antigène de contrôle fixé sur ledit support solide.

La spécificité de la réaction antigène infectieux/anticorps sérique conditionne la spécificité et la valeur prédictive positive du test sérologique basé sur cette réaction et toute fixation d'un anticorps non-spécifique sur l'antigène microbien limite la spécificité et la valeur prédictive du test sérologique. La présence dans le sérum à tester, d'anticorps anti-nucléaires, est une source de fixation non-spécifique d'anticorps sur l'antigène microbien comme expliqué ci-après.

La présence dans le sérum du patient d'anticorps anti-nucléaires limite la spécificité de la réaction antigène microbien-anticorps sériques. Les anticorps anti-nucléaires sont en effet des anticorps du type IgG dirigés de façon non-spécifique contre l'ensemble formé par l'ADN et les protéines nucléaires du chromosome des cellules eucaryotes et des microbes, appelées histones. Ces anticorps anti-nucléaires se fixent donc de façon non-spécifique sur toute cellule eucaryote y compris les champignons et les parasites et sur tout microbe, bactérie, virus à ADN, parasite ou champignon. Ce phénomène entraîne une réaction positive non-spécifique au cours des tests sérologiques utilisant la détection d'IgG spécifique d'une bactérie, un virus à ADN, un champignon ou un parasite entier ou comprenant des complexes ADN/histones comme antigène microbien à l'aide d'anticorps de détection anti IgG dans un sérum contenant des anticorps anti-nucléaires.

La détection des anticorps anti-nucléaires dans le sérum à tester, réalisée en utilisant une technique d'immunodétection par immunofluorescence, a été décrite [Fritzler M.J. In : Manual of Clinical Laboratory Immunology, Fourth edition, Rose N.R., Conway de Macario E., Fahey J.L., Friedman H., Penn, G.M., (eds.). American Society for Microbiology, Washington, D.C. 1992, pp. 724-729] utilisant des cellules mammifères y compris des cellules humaines telles que des cellules Hep-2 comme antigène. Dans ces conditions, la prévalence des anticorps antinucléaires détectés sur le sérum pur est de 2% dans la population générale, de 20% parmi les parents de patients présentant une polyarthrite rhumatoïde, et de 75% parmi les personnes âgées sans pathologie clinique apparente [Mc Carty G.A., and coll. Antinuclear antibodies : contemporary techniques and clinical applications to connective tissue diseases. Oxford University Press, New-York, 1984]. Dans cette méthode, les cellules sont constituées par un tapis de cellules confluentes déposées et « lues » manuellement. Ce dépôt de cellules confluentes est trop visqueux pour être robotisable par dépôt avec un robot de type seringue, et la lecture n'est pas automatisable car, seul, le dépôt manuel permet de déposer une grande quantité de cellules de manière à garantir une détection suffisante de la réaction Cellule-anticorps anti-nucléaires. Dans ces publications, on ne mentionne pas la nécessité de réaliser un contrôle systématique de la présence d'anticorps anti-nucléaires dans des tests d'immunodétection d'antigènes microbiens. En outre, la détection avec des cellules confluentes déposées manuellement sur support solide n'est pas applicable pour des tests de routine de laboratoire.

Il n'y a donc pas de méthode publiée à ce jour, pour le contrôle ou l'élimination des anticorps anti-nucléaires avant la réalisation d'un test sérologique pour le diagnostic des maladies infectieuses de façon à garantir lors du résultat l'absence d'une réaction faussement positive applicable en routine de laboratoire. C'est pourquoi, aucun protocole de sérologie publié dans les manuels de référence ni aucun test sérologique commercialisé n'inclut systématiquement le dépistage d'anticorps anti-nucléaires comme préalable à la réalisation ou à l'interprétation de la sérologie.

En pratique, à ce jour, dans les tests d'immunodétection en routine de laboratoire, on ne peut, tout au plus, détecter les faux positifs dus à la présence d'anticorps anti-nucléaires qu'en effectuant des tests sur une pluralité d'antigènes microbiens dont la présence concomitante n'est pas vraisemblable. Mais, ce type de vérification, on le comprend, augmente considérablement le coût en terme de matériel et main d'oeuvre ainsi que la durée des tests.

Pour ce faire, la présente invention fournit une méthode dans laquelle, on contrôle la présence éventuelle d'un anticorps anti-nucléaire dans ledit échantillon à tester en
- mettant en contact ledit échantillon à tester avec
   - un support solide sur lequel a été fixé un deuxième antigène de contrôle qui comprend des complexes ADN/histones, de préférence comprenant des noyaux de cellules nucléées de cellules de l'espèce du patient, de préférence encore toute ou partie de cellules d'origine de l'espèce du patient en lignée continue, et
   - en présence d'une dite substance de détection constituée par un anticorps de marquage qui ne réagit qu'avec une dite immunoglobuline de l'espèce du patient de classe G; et
- en vérifiant si ledit deuxième antigène de contrôle fixé sur le support solide réagit avec ladite substance de détection.

Dans le cas d'un patient humain, on peut, notamment, utiliser des cellules de fibroblastes humains non confluentes en suspension, notamment des cellules HL60.

Si l'échantillon à tester comprend des anticorps anti-nucléaires (qui sont des IgG, notamment humaines), ceux-ci peuvent réagir avec ledit deuxième antigène (Ag₂) et être détectés par ladite substance de détection (Ac₁ anti IgG*) puisque celle-ci est une substance réagissant avec des IgG de l'espèce du patient, notamment humaines, et former le complexe (S-Ag₂-IgG anti Ag₂- Ac₁ anti IgG*).

Une fois l'absence d'anticorps anti-nucléaire établie, la détection d'une réaction de ladite substance de détection avec ledit antigène vaccinal microbien est bien la preuve de la présence d'IgG spécifique du dit antigène vaccinal microbien et de formation d'un complexe (S-Agvac-IgG antiAgvac-Ac₁ anti IgG*) et non pas d'un complexe faux positif résultant de la réaction des anticorps anti-nucléaires avec l'antigène microbien selon le complexe (S-Agvac-Ac anti nucl.-Ac₁ anti IgG*).

De préférence encore, on vérifie la réactivité de ladite substance de détection introduite dans ledit échantillon de sérum à tester, avant de vérifier l'absence d'anticorps anti-nucléaires dans ledit échantillon.

Si ladite substance de détection est bien réactive, on doit détecter le complexe suivant (S-IgG₁-Ac₁ anti IgG*) avec ledit premier antigène (IgG₁). Dans ce cas, l'absence de réaction de ladite substance de détection avec ledit deuxième antigène est bien la preuve de l'absence d'anticorps anti-nucléaires.

Plus particulièrement, le dépôt d'IgG de l'espèce du patient, notamment humaines, γ-spécifique (spécifique de la chaîne gamma des immunoglobulines de l'espèce du patient, notamment humaines) comme dit premier antigène permet de contrôler positivement l'introduction de l'immunoglobuline conjuguée anti-IgG au cours de la réaction sérologique ainsi que sa réactivité immunologique (aspect qualitatif). En effet, cet anticorps conjugué va se fixer également sur le dépôt d'IgG qui sera donc reconnu obligatoirement au cours de la phase de détection des IgG spécifiques de la réaction sérologique.

La présente invention permet donc la détection systématique de d'anticorps anti-nucléaires, dans un sérum utilisé pour un diagnostic sérologique par immunofluorescence indirecte, après le dépôt robotisé d'immunoglobulines de l'espèce du patient, notamment humaines, de classe G (IgG) et de cellules nucléées de l'espèce du patient, notamment humaines, sur le support de la réaction sérologique. De même, le dépôt robotisé d'immunoglobulines de l'espèce du patient, notamment humaines, IgG permet de contrôler la présence des anticorps secondaires anti-immunoglobulines G de l'espèce du patient, notamment humaines, mis en oeuvre.

Une erreur fréquente dans la réalisation des tests sérologiques, notamment pour les tests sérologiques en batterie réalisés sur un grand nombre de sérums à tester, est due à des défauts dans l'introduction des sérums à tester, notamment par pipetage. Ces erreurs interviennent notamment dans les étapes qui impliquent le déplacement de l'échantillon à tester, notamment par pipetage, certains récipients, notamment contenant le support solide sur lequel est déposé l'antigène à détecter, peuvent ne pas être remplis par inadvertance avec le sérum de l'espèce du patient, notamment humaine, à tester. Il est connu que le pipetage de sérum est entaché d'un risque d'erreurs de 1‰, liée à un problème purement technique par absence de pipetage par la pipette, ou à une erreur humaine par absence de pipetage par inadvertance.

Ces erreurs imposent l'introduction de contrôles dans la réalisation de la réaction. L'incorporation systématique au cours de chaque nouvelle manipulation d'un sérum témoin négatif c'est à dire ne contenant pas d'anticorps spécifiques de l'antigène à tester permet d'interpréter les réactions positives. De même, l'incorporation d'un sérum témoin positif, c'est à dire contenant l'anticorps spécifique de l'antigène testé à un titre connu permet de vérifier la qualité de l'antigène et de l'immunoglobuline conjuguée.

Cependant, il n'existe actuellement aucun contrôle fiable de ce que le sérum à tester a bien été introduit dans le test sérologique. Or, si par inadvertance, le sérum à tester n'est pas introduit dans le test sérologique, la réaction antigène bactérien-anticorps sérique n'existera certainement pas, et le test sera interprété, faussement, comme négatif (faux-négatif). Dans la présente invention, on tire partie de ce que la protéine A du *Staphylococcus aureus* (staphylocoque doré) présente une affinité pour les sérums d'origine animale, notamment le cheval, les bovins, le cochon, le lapin, le cobaye, la souris ; à un moindre degré le hamster, le rat et le mouton. En revanche, le sérum de poussin et de chèvre ne réagissent pas avec la protéine A. La protéine A est un polypeptide de 42 kDa, et est un constituant de la paroi des souches de *Staphylococcus aureus,* des protéines similaires mais différentes sont caractérisées à la surface des bactéries du genre *Streptococcus* [Langone J.J. Adv. Immunol. 1982, 32 : 157-251]. Cette propriété de la protéine A de *Staphylococcus aureus,* a déjà été utilisée dans un test sérologique chez l'homme pour le diagnostic sérologique des endocardites infectieuses [Rolain JM, Lecam C, Raoult D. Simplified serological diagnosis of endocarditis due to Coxiella burnetii and Bartonella. Clin. Diag. Lab. Immunol. 2003 ; 10 :1147-8].

La présente invention comprend donc l'introduction d'un contrôle d'introduction du sérum à tester au cours des réactions de sérologies.

Pour ce faire, qu'on contrôle que ledit échantillon testé contient bien un sérum de l'espèce du patient, en détectant si des immunoglobulines de l'espèce du patient réagissent avec un troisième antigène de contrôle contenant la protéine A d'une bactérie *Staphylococcus aureus,* de préférence en mettant en contact ledit échantillon avec un même dit support solide sur lequel est fixé un dit troisième antigène de contrôle, en présence d'une même dite substance de détection qui est une substance réagissant avec une immunoglobuline de l'espèce du patient et pas avec ledit troisième antigène de contrôle, de préférence un anticorps anti-immunoglobuline de l'espèce du patient et ne réagissant pas avec la protéine A.

Dans la mesure où la protéine A réagit avec des immunoglobulines animales et humaines de façon non spécifique, et ce même en cas de pathologie infectieuse importante, il est possible d'utiliser cette protéine A comme contrôle positif de l'introduction d'un sérum de l'espèce du patient, notamment humaine, dans l'échantillon à tester.

Dans un mode de réalisation avantageux, ledit troisième antigène de contrôle est une bactérie entière *Staphylococcus aureus* comprenant la protéine A. On peut plus particulièrement utiliser les bactéries *Staphylococcus aureus* déposées dans les collections publiques telles que les bactéries déposées à l'A.T.C.C. sous le N°29213 et à la C.N.C.M. de l'Institut Pasteur (France) sous le numéro 65.8T comme décrit dans la publication mentionnée ci-dessus [Rolain JM, Lecam C, Raoult D. Simplified serological diagnosis of endocarditis due to Coxiella burnetii and Bartonella. Clin. Diag. Lab. Immunol. 2003 ; 10:1147-8].

Par ailleurs, en dehors des souches-types, toute souche bactérienne identifiée comme *Staphylococcus aureus* peut être utilisée comme dit troisième antigène de contrôle.

De préférence, ladite substance de détection est une immunoglobuline animale, de préférence encore une immunoglobuline de chèvre ou de poulet.

Avantageusement, on réalise successivement les étapes de :
- contrôle de la présence d'un sérum dans l'échantillon à tester en présence de ladite substance de détection, et
- contrôle de la présence d'anticorps anti-nucléaire dans l'échantillon à tester.

Avantageusement encore, on détecte et, le cas échéant, on quantifie la dose de dite immunoglobuline de l'espèce du patient de classe G spécifique dudit antigène vaccinal dans l'échantillon à tester, par lecture automatisée de l'intensité du signal émis par ledit élément de marquage à l'aide d'un appareil de lecture approprié audit signal par ledit élément de marquage.

Comme élément de marquage des dites substances de détection, on peut aussi utiliser un élément de marquage radioactif, bien que les marquages fluorescents restent préférés, comme mentionné précédemment.

L'expression "marquage radioactif" signifie que l'anticorps porte un isotope radioactif permettant de le doser par comptage de la radioactivité qui lui est associée, l'isotope pouvant être porté soit sur un élément de la structure de l'anticorps, par exemple les résidus de tyrosine constitutifs, soit sur un radical approprié qui lui a été fixé.

Lorsqu'on utilise une sonde radioactive, comme par exemple l'iode 125, la radioactivité associée à l'échantillon testé est complétée dans un compteur gamma selon toute modalité appropriée et par exemple après solubilisation des cellules par une solution alcaline (par exemple une solution de soude) et récupération de la solution contenant la radioactivité à l'aide d'un tampon absorbant.

Dans un mode de réalisation, un protocole de succession de lecture des contrôles, est le suivant :
1) On vérifie que ledit troisième antigène de contrôle contenant la protéine A réagit avec une dite substance de détection. A défaut, on arrête le test, c'est-à-dire on ne prend pas en compte cet échantillon.
2) Si ledit premier antigène de contrôle (IgG₁) ne réagit pas avec ladite substance de détection (Ac₁ anti IgG^{*}), ladite substance de détection n'est pas présente ou pas réactive. On ne prend pas en compte le résultat du test concernant la détection des IgG spécifiques de l'antigène microbien.
3) Si ledit premier antigène de contrôle (IgG₁) réagit avec la substance de détection, on peut continuer le test, c'est-à-dire prendre en compte les résultats sous réserve des vérifications suivantes concernant les réactions avec les antigènes de contrôle.
4) Si ledit deuxième antigène de contrôle contenant un complexe ADN/histone réagit avec ladite substance de détection, des anticorps antinucléaires sont présents et on ne prend pas en compte les tests de détection et de quantification des IgG spécifiques.
5) Si ledit deuxième antigène ne réagit pas, et que ladite substance de détection est présente et réactive, il n'y a pas d'anticorps anti-nucléaire, et on peut continuer sous réserve de la vérification suivante.

En résumé, on ne prend en compte le résultat de la réaction avec ledit antigène vaccinal microbien, que si les conditions cumulatives suivantes sont réunies :
- ledit premier antigène de contrôle (IgG) réagit avec ladite substance de détection, et
- ledit deuxième antigène de contrôle (ADN) ne réagit pas, et
- ledit troisième antigène de contrôle (protéine A) réagit avec ladite substance de détection.

Dans un mode préféré de réalisation, pour chaque détection et, le cas échéant, quantification d'un dit antigène vaccinal, on réalise les mesures suivantes :
1- une première mesure d'une première valeur représentative de la quantité d'un premier élément de marquage de préférence la première valeur de l'intensité d'un signal émis par ledit premier élément de marquage fluorescent, ledit premier élément de marquage se fixant de manière non spécifique sur toute protéine dans la zone de dépôt dudit antigène vaccinal, et
2- une deuxième mesure d'une deuxième valeur représentative de la quantité d'un deuxième élément de marquage différent dudit premier élément de marquage et émettant un signal différent, de préférence une deuxième valeur de l'intensité du signal émis par ce deuxième élément de marquage fluorescent à une longueur d'onde d'excitation différente de celle dudit premier élément de marquage fluorescent, ledit deuxième élément de marquage étant l'élément de marquage de ladite substance de détection dudit antigène vaccinal, dans la zone de dépôt dudit antigène, et
3- on calcule le rapport desdites première et deuxième valeurs, et
4- on compare la valeur dudit rapport à celle d'un rapport de référence obtenu avec une collection de sérums de référence positifs et négatifs, permettant ainsi par comparaison de déterminer la nécessité ou non de vacciner la personne pour ledit antigène vaccinal selon la valeur du rapport desdites première et deuxième valeurs.

Une trousse de diagnostic utile pour la mise en oeuvre d'une méthode selon l'invention, comprend :
- un dit même support solide sur lequel est fixé au moins une dite pluralité d'antigènes vaccinaux et le cas échéant au moins un dit antigène de contrôle, et
- des réactifs tels qu'une dite substance de détection et si nécessaire des réactifs utiles pour la détection dudit élément de marquage.

De préférence, la trousse comprend :
- un même dit support solide sur lequel sont fixés au moins un dit antigène vaccinal corpusculaire et lesdits premiers ,deuxième et troisième antigènes de contrôle, et
- une même dite substance de détection.

Avantageusement, dans les méthodes de diagnostic selon l'invention, on utilise comme support solide une lame de verre ou en plastique, ou un puits d'une plaque de microtitration à fond plat, en verre ou en plastique.

Pour la mise en oeuvre de la détermination du statut vaccinal selon la présente invention, il est possible de prélever le sérum par une ponction veineuse à l'aiguille ou bien par prélèvement de sang capillaire, notamment à partir du lobe de l'oreille, de la pulpe du doigt, du talon, couplé à un recueil sur un disque de papier filtre ou de préférence directement dans un tampon permettant l'élution du sérum.

Selon une autre caractéristique avantageuse de la présente invention, on réalise, après un prélèvement de sang capillaire dans un tube capillaire permettant de recueillir un volume connu de sang total, le recueil dudit échantillon dans un flacon contenant un volume connu d'un tampon d'élution. Cette modalité de recueil originale présente comme avantages sa rapidité (les recueil, élution, et dilution se faisant en une minute). Le fait que le sérum est dilué à une concentration connue, notamment de 1/20 à 1/100, est une sécurité de manipulation vis à vis du risque d'accidents d'exposition au sang tels que transmission de virus et autres pathogènes sanguins pour le préleveur.

Par ailleurs, la présente invention permet la détermination du statut vaccinal de la personne contre plusieurs simultanément contre plusieurs antigènes vaccinaux, sur un seul échantillon de sérum prélevé par ponction capillaire, dans un laps de temps inférieur à 2 heures.

Avantageusement, on recueille un volume déterminé de sang total à l'aide d'un tube capillaire dans un flacon contenant un volume déterminé d'un tampon permettant l'élution du sérum, le sérum étant alors de préférence dilué à une concentration déterminée, de préférence de 1/100 à 1/20, et l'on dépose un volume déterminé de sérum ainsi dilué sur les différentes zones de dépôt desdits antigènes de contrôle et antigènes vaccinaux sur ledit support solide.

Quel que soit le mode de recueil du sang, « prise de sang » ou « ponction capillaire », il convient dans un premier temps de séparer le sérum des globules sanguins .Dans le cas de la ponction capillaire, cette séparation est réalisée par une élution par un tampon d'élution, et c'est le produit de cette élution qui est mis en contact avec la lame.

Et avantageusement donc, la trousse comprend un flacon comportant un volume déterminé d'un tampon d'élution pour le recueil d'un volume déterminé d'un échantillon de sérum à tester.

On connaît des méthodes et kit de diagnostic impliquant l'utilisation d'un support solide sur lequel sont fixés de façon covalente des protéines solubles, mais ces couplages chimiques covalents sont complexes et coûteux à réaliser. On a proposé la fixation non covalente de protéine soluble ou antigène particulaire sur support solide par adsorption physique ou physico-chimique sur le support, dans le cadre des protocoles de tests d'immunodétection sur support solide, mais la stabilité de la fixation est insuffisante. Une difficulté tient à ce qu'il est nécessaire de bien laver préalablement le support solide pour éliminer les résidus d'éléments de marquage pouvant interférer avec la lecture des résultats alors que ces lavages rendent l'adsorption physique des substances sur le support solide trop instable. Une autre difficulté tient à ce qu'il n'est pas possible de déposer, avec des robots de dépôts, des antigènes corpusculaires tel quels, qu'il s'agisse de cellules ou bactéries entières ou partielles comme mentionné ci-dessus.

Un autre but de la présente invention est donc de fournir un test fiable d'immunodétection d'antigènes vaccinaux microbiens, notamment d'antigènes vaccinaux viraux particulaires, par une méthode et des outils simples à mettre en oeuvre et réaliser pour une application en routine de laboratoire dans le cadre de la réalisation des tests en série notamment.

Un autre but de la présente invention est de fournir une méthode de préparation de support solide permettant un dépôt robotisé sur le support, d'antigènes de contrôle ou vaccinaux particulaires ou corpusculaires, ainsi que la lecture automatisée des résultats de réaction immunologique desdits antigènes fixés sur support solide dans un test de sérum de patient impliquant une réaction de sérologie microbienne des IgG spécifiques par adsorption avec l'antigène vaccinal ou antigène de contrôle particulaire ou corpusculaire déposé sur support solide.

Une méthode de préparation d'un support solide utile dans une méthode ou une trousse selon l'invention, est caractérisée en ce que l'on dépose et fixe par adsorption physique sur ledit support solide une pluralité de dits antigènes vaccinaux comprenant au moins un antigène vaccinal corpusculaire sous forme de suspension de microbes entiers ou fraction de microbes, notamment virus ou bactéries, de préférence, et le cas échéant, un antigène vaccinal sous forme de virus entier vivant désactivé, et, le cas échéant, des dits antigènes de contrôle sous forme de suspension de corpuscules de cellules non confluentes ou bactéries entières ou fraction de cellules ou bactéries, lesdits antigènes corpusculaires étant de préférence déposés par un robot de dépôt comprenant notamment du type seringue.

De préférence, lesdits antigènes corpusculaires vaccinaux et/ou de contrôle, sont associés à un colorant, de préférence un colorant fluorescent, sous forme de suspension à une concentration permettant leur visualisation après dépôt à l'aide dudit colorant, permettant de vérifier la fixation desdits antigènes sur le support solide.

Les inventeurs ont mis au point, après de nombreux essais infructueux, des conditions de dépôt robotisé d'antigènes vaccinaux microbiens corpusculaires (microbe entier inactivé ou fraction de microbe entier inactivé) en suspension dans un milieu de dépôt. En effet, actuellement, on dépose sur support solide, par les robots de dépôt, uniquement des solutions homogènes d'une ou de plusieurs molécules. Pour ce faire, la concentration de ces antigènes corpusculaires est calibrée avant dépôt par comptage des particules microbiennes inactivées par « fluorescence activated cell sorting (FACS-scann) puis déposés de façon robotisée sur un support solide. La mise au point de ces dépôts calibrés et robotisés comporte la détermination par des essais, de la concentration optimale pour chacun des antigènes testés, les concentrations infra-optimales donnant des dépôts indétectables, les concentrations supra-optimales entraînant une sédimentation des antigènes corpusculaires de forte densité et de dimensions micrométriques tels que les bactéries entières ou fractionnées en cours de dépôt et donc une variation sensible de la quantité d'antigène déposé. Enfin, les dépôts d'antigènes corpusculaires comportant de l'ADN microbien (bactérie, virus, parasites ou champignons microscopiques sont calibrés par application d'un colorant de préférence fluorescent, notamment une molécule comme l'AMCA qui se fixe de façon non spécifique aux protéines contenues dans l'antigène ou une molécule intercalante qui se fixe de façon non spécifique à l'ADN par intercalation dans la double hélice. Cette dernière méthode est utilisée de préférence pour marquer les cellules utilisées comme témoin sur les lames. Les longueurs d'onde d'excitation et d'émission sont avantageusement choisies en fonction de celles utilisées par le fluorochrome marquant les immunoglobulines de détection. Ce marquage fluorescent, non spécifique des antigènes peut être réalisé avant leur dépôt par le robot de dépôt ou après celui-ci. Le marqueur fluorescent est avantageusement choisi pour sa stabilité à la lumière du jour.

Plus particulièrement, dans la méthode de préparation de support solide, les antigènes de contrôle sous forme de suspension de cellules sont calibrés à une concentration de 10⁷ à 10⁹ cellules/ml, les suspensions de bactéries ou fractions de bactéries à une concentration de 10⁷ à 10⁹ particules/ml et les suspensions de virus entiers à une concentration de 10⁹ à 10¹⁰ particules/ml.

Avantageusement, on dépose lesdits antigènes de contrôle et vaccinaux corpusculaires en mélange avec un liant protéique, stabilisant la fixation sur ledit support solide.

Plus particulièrement, ledit liant protéique est choisi parmi le mélange organique complexe formé par le jaune d'oeufs, de la gélatine, de l'albumine de sérum de bovin ou une IgG polyclonale non humaine, de préférence de chèvre.

Ces liants protéiques fonctionnent comme une colle biologique dudit antigène sur le support solide;

Les différents liants ont été testés sur lame de verre et on a déterminé les concentrations optimales de mise en oeuvre. On peut notamment utiliser l'albumine sérique bovine à une concentration finale (volume pour volume) de 1 à 5%, une suspension de jaune d'oeuf à une concentration finale de 1 à 10% et ladite IgG caprine à une concentration finale de 25 à 75%.

Les inventeurs ont remarqué au cours de différents essais réalisés, que certains antigènes liés par de l'oeuf ou de l'albumine bovine étaient lavés lors des étapes de lavage mais que les immunoglobulines humaines IgG introduites comme contrôle restaient invariablement fixées sur la lame de verre. Les inventeurs en ont déduit que les immunoglobulines de classe G se fixaient à la lame de verre d'une façon telle qu'elles supportaient les étapes de lavage et ont formé l'hypothèse que ces IgG permettraient donc de fixer également sous des conditions appropriées certains antigènes vaccinaux. Les inventeurs ont donc utiliser des IgG d'une autre espèce que l'homme, afin de ne pas interférer dans les tests sérologiques et les auteurs ont ainsi déterminé les propriétés remarquables des IgG de chèvre à titre de colle biologique permettant de fixer les antigènes particulaires, notamment les antigènes vaccinaux particulaires ou corpusculaires.

De préférence et plus particulièrement, ledit antigène vaccinal corpusculaire est déposé sur ledit support solide constitué d'une lame de verre, en mélange avec une immunoglobuline de type IgG polyclonale de chèvre.

Dans une méthode de préparation d'un support solide sur lequel est fixé une pluralité de dits antigènes vaccinaux, et le cas échéant, desdits premier, deuxième et troisième antigènes de contrôle, permettant une détection par lecture automatisée à l'aide d'une dite substance de détection, utile dans une méthode selon l'invention, on réalise un lavage préalable du dit support solide avec une solution d'un mélange éthanol/acétone, de préférence à 50/50, puis on dépose et on stabilise la fixation des dits antigènes par adsorption physique sur ledit support solide par un traitement avec de l'alcool, de préférence méthanol ou éthanol, alcool que l'on élimine ensuite et, de préférence, on vérifie la fixation des dits antigènes par coloration, notamment par un marquage fluorescent non spécifique des protéines ou de l'ADN tel qu'explicité ci-dessus.

Cette solution de prélavage permet de nettoyer le support de toute trace de substance de détection ou autre résiduelle et, notamment, éliminer toute fluorescence, tout en conservant la faculté d'adsorption physique du support vis à vis des dits antigènes déposées ensuite.

Le traitement de stabilisation à l'alcool permet de stabiliser la fixation par adsorption physique aussi bien des protéines, telles que IgG, que des antigènes particulaires.

La détermination du lavage préalable approprié du support solide, notamment de la lame de verre a donné lieu à de nombreux essais. Ce traitement a pour objectif de nettoyer parfaitement ce support pour éliminer les artéfacts fluorescents tout en préservant la fixation ultérieure des antigènes de façon compatible avec leur mode de conservation mais, aussi, en préservant sinon l'intégrité au moins la réactivité immunologique des antigènes. Il a été montré, après de nombreuses recherches infructueuses, que le rinçage et le nettoyage des lames en phase aqueuse ne permettraient pas une fixation ultérieure des antigènes à déposer ; il en allait de même pour le rinçage à base de molécules tensio-actives comme le Tween 20. Le nettoyage avec des alcools n'était pas suffisant pour retirer la plupart des artéfacts fluorescents. C'est donc au terme de ces multiples essais qu'a été optimisé un protocole de nettoyage de support solide, notamment de la lame de verre, par un mélange éthanol 50 % - acétone 50 % puis séchage à l'air.

Toutefois, même dans ce cas, il reste avantageux de compléter la fixation par adsorption physique par un traitement de réticulation, notamment par traitement chimique avec un agent bi-fonctionnel de couplage covalent tel que le glutaraldéhyde ou des dérivés diacides activés, notamment de l'acide succinique, connus de l'homme de l'art pour assurer des pontages covalents entre lesdits antigènes de contrôle et microbiens avec le support solide.

D'autres caractéristiques et avantages de la présente invention apparaîtront à la lumière des exemples qui vont suivre.

### Exemple 1 : Dépôt de dits deuxième et troisième antigènes de contrôle sur support solide.

Les cellules HL60 (N° ATCC CCL 240) sont des cellules fibroblastiques humaines en lignée continue utilisées pour la détection des anticorps anti-nucléaires. Après culture et production selon les protocoles habituels, on a quantifié la concentration des cellules HL 60 à l'aide d'un compteur de cellules (Microcytes® BPC / Yeast, BioDETECT AS, Olso, Norvège) et cette concentration a été rapportée à 10⁸ cellules / mL dans un tampon BPS stérile, pH= 7,4 pour obtenir une suspension de cellules non confluentes pouvant être déposées par robot de dépôt.

*Staphylococcus aureus* (n° de dépôt ATCC 29213) a été cultivé sur gélose à 5% de sang de mouton, puis récolté dans un tampon PBS stérile contenant 0,1% d'azide de sodium. L'inoculum a été mesuré à l'aide du même compteur de cellules et calibré à 10⁹ bactéries/mL qui est la concentration optimum compte tenu des contraintes d'absence de sédimentation en cours de dépôt et d'un dépôt en quantité suffisante de particules staphylococciques puis conservé par congélation à - 80°C.

On a déposé ces cellules HL60 et des bactéries *Staphylococcus aureus* à une concentration de 10⁹ CFU/mL déterminée par FACS-scan (Microcytes®) des lames en verre (Référence LLR2-45, CML, Nemours, France). Les cellules et les bactéries ont été déposées sur le support solide à l'aide d'un robot de dépôt ou " spotter" (Arrayer 427 ^{™}, Affymetrix, MWG Biotech SA, Courtaboeuf, France). Les dépôts ont été séchés à l'air pendant 30 minutes dans l'enceinte du "spotter", puis fixés dans un bain de méthanol à 100 % pendant 10 minutes, puis séchés à nouveau à l'air libre. L'efficacité du dépôt robotisé sur les lames, après la fixation à l'éthanol et après les bains nécessités par la réaction d'immunofluorescence indirecte suivante, a été vérifiée avec succès par la coloration par le colorant fluorescent Hoescht 333-42 (molécule intercalante qui s'intercale dans l'ADN) qui est excité à 350 nanomètres et qui émet à 460 nanomètres (Molecular Probes).

### Exemple 2 : dépôt de dit premier antigène de contrôle (IgG) sur support solide

Des immunoglobulines humaines de classe G γ-spécifiques (IgG) (Serotec, Cergy Saint-Christophe, France) diluées à une concentration de 5mg/mL pour obtenir des "spots" bien homogènes, ont été déposées de façon robotisée à l'aide d'un robot de dépôt (modèle 427, Arrayer, Affymetrix, Inc., CA) sur un support solide constitué d'une lame de verre (Référence LLR2-45, CML, Nemours, France).

Les dépôts ont été réalisés par transfert de la suspension d'antigène d'un puit de plaque de microtitration contenant 25 µL de suspension, sous un volume de 1 mL déposé, à 25°C et 55 % d'humidité dans l'enceinte du robot de dépôt. Ces conditions ont été contrôlées par un thermo-hygromètre. Les dépôts d'une dimension de 200 µm ont été séchés à l'air pendant 30 minutes à 37°C dans l'enceinte du robot de dépôt, puis fixés dans un bain d'éthanol à 100% pendant 10 minutes, puis séchés à nouveau à l'air libre. L'efficacité du dépôt robotisé puis de la fixation à l'éthanol après les bains nécessités par la réaction d'immunofluorescence indirecte a été vérifiée par la technique d'immunofluorescence indirecte.

10 sérums humains ont été déposés chacun sur un dépôt d'IgG sous trois dilutions chacun, 1 :32, 1 :64 et 1 :128. Après un premier lavage, une réaction d'immunofluorescence indirecte a été réalisée en utilisant une immunoglobuline de chèvre (référence A-21216, Molecular Probes, Eugene, USA) à titre d'anticorps secondaire anti-IgG humaines, couplée à l'Alexa 488 qui est une molécule fluorescente excitée à 488 nanomètres et émettant à 540 nanomètres et en utilisant la même immunoglobuline anti-IgG couplé à l'Alexa 594 (A-21216, Molecular Probes, Eugene, USA) qui est une molécule fluorescente excitée à 594 nanomètres et émettant à 640 nanomètres dans une deuxième expérience. La lecture de la réaction a été faite au microscope à fluorescence et a montré une détection fluorescente dans tous les sérums sous forme d'un "spot" très brillant pour chacune des 3 dilutions testées. Cet exemple illustre qu'il est possible de réaliser un dépôt robotisé d'IgG humaines sur un support solide dans des conditions compatibles avec la réalisation d'une réaction d'immunofluorescence indirecte.

### Exemple 3 : Détermination du statut vaccinal de personnes.

On a mis au point une lame de verre pour la détermination du statut vaccinal des personnes comportant un total de 8 dépôts d'antigènes comportant 3 dépôts de contrôles et 5 dépôts d'antigènes vaccinaux sur une même lame, répartis en 2 colonnes. Les modalités de dépôt et d'utilisation des 3 dépôts contrôles comportant Staphylococcus aureus, IgG et cellules HL-60 ont été exposes dans les exemples 1 et 2 ci-dessus.

Les dépôts d'antigènes vaccinaux ont été réalisés sous forme de suspension virale de virus entiers vivants inactivés déposés à une concentration de 10⁹ ou 10¹⁰ particules/ml en mélange avec une IgG caprine à une concentration de 25 à 75% (volume pour volume) et suivant les modalités suivantes :
(1) antigène rubéole : il s'agit de la souche hpv-77 (microbix Biosystems, Inc., Toronto, Ontario, Canada) fournie sous forme d'une suspension virale inactivée à concentration protéique de 0, 51 mg/ml. Cet antigène a été concentré 10 fois par centrifugation avant son dépôt sous un volume de 45 µL d'antigène et 5 µL d'immunoglobulines IgG de chèvre titrant à 10 mg/ml (référence I5256, Sigma, Saint-Quentin Fallavier, France), colorées par 2 µL d'amino methyl coumarin acetyl (AMCA) (Molecular Probes, Interchim, Montluçon, France).
   L'AMCA se fixe de façon non spécifique aux protéines de cet antigène et permet donc de vérifier le dépôt de l'antigène sur le support solide par adsorption physique. Il est excité à une même longueur d'onde de 350 nm que le colorant HOESCHT 333-42.
(2) antigène rougeole : il s'agit de la souche Edmonston (Microbix Biosystems, Inc.) fournie sous forme d'une suspension de particules virales inactivées à une concentration protéique de 1,95 mg/ml qui a été concentrée 5 fois par centrifugation avant son dépôt sous un volume de 45 µL d'antigène et 5 µL d'immunoglobuline IgG de chèvre colorée à l'AMCA.
(3) Antigène oreillons : il s'agit de la souche Enders (société microbix-biosystem incorporated) titrant à une concentration protéique de 1,8 µg/ml et déposée sous un volume de 45 µL d'antigène et 5 µL d'immunoglobiline IgG de chèvre marqués à l'AMCA.
(4) anatoxine diphtérique (référence FA 150934, Aventis Pasteur Vaccins) titrant à une concentration protéïque de 18,9 mg/ml et concentrée 10 fois avant son dépôt sous un volume de 45 µL d'antigène et 5 µL d'immunoglobulines IgG de chèvre marquées à l'AMCA,
(5) anatoxine tétanique (référence J001, Aventis Pasteur vaccins) titrant à une concentration de 80 Ul/mL et déposée pure sous un volume de 45 µL d'antigène et 5 µL d'immunoglobuline IgG de chèvre marquées à l'AMCA.

Les dépôts d'antigènes ont été réalisés sous un volume de 1 nl par un "spotter" de marque Affymetrix®, de modèle Arrayer 427. Après séchage, les lames ainsi préparées ont servi de support à une réaction d'immunofluorescence indirecte pour la détection des IgG spécifiques des antigènes vaccinaux dans le sérum des personnes selon le protocole suivant :
1- dans une première étape, 5 µL de sérum prélevé par ponction veineuse ou par ponction capillaire, sont mis en contact et incubés avec la lame au niveau des différents antigènes vaccinaux et antigènes de contrôles, par un automate d'incubation.
2- dans une deuxième étape, l'automate rince la lame afin d'éliminer le sérum testé et réalise une incubation avec l'anticorps de détection anti-IgG humaines conjugué à la fluorescéine qui est excitée à une longueur d'onde de 488 nm (référence Star 106 F, Serotec, France),
3- dans une troisième étape, l'automate rince la lame afin d'éliminer l'anticorps de détection conjugué, et sèche la lame,
4- dans une quatrième étape, la lame ainsi traitée est retirée de l'automate d'incubation et placée dans la chambre de lecture d'un lecteur automatique de fluorescence. Ce lecteur réalise successivement deux lectures, une à 350 nm qui est la longueur d'onde d'émission du colorant AMCA puis une deuxième lecture à 488 nm qui est la longueur d'onde d'émission de la fluorescéine de l'anticorps de détection,
5- dans une cinquième étape, ces données sont transmises à un logiciel afin d'être converties en intensité de fluorescence à 350 nm et à 488 nm pour chacun des dépôts,
6- dans un sixième étape, le logiciel analyse les niveaux de fluorescence des contrôles et vérifie successivement
   . la présence de fluorescence pour le dépôt *Staphylococcus aureus* pour vérifier la présence du sérum à tester ;
   . la présence de fluorescence du dépôt d'IgG pour vérifier la qualité de la réaction impliquant l'anticorps de détection conjugué ;
   . l'absence de fluorescence du dépôt de cellules HL60 pour vérifier la présence ou l'absence d'anticorps anti-nucléaires dans le sérum à tester.
7- dans une septième étape, le logiciel calcule le ratio de fluorescence 488/350 pour chacun des dépôts d'antigènes vaccinaux et compare pour chaque dépôt d'antigène vaccinal cette valeur à une courbe de ratio préalablement établie pour chaque antigène vaccinal à l'aide de sérums témoins positifs possédant des anticorps spécifiques à une concentration connue par une méthode de référence et de sérums négatifs ne possédant pas d'anticorps spécifiques détectables par une méthode de référence,
   La fluorescence à 350 nn qui résulte de l'excitation de marqueurs non-spécifiques qui se fixent de façon non-spécifique soit à l'ADN soit aux protéines des antigènes vaccinaux. La quantité de fluorescence émise par les dépôts d'antigènes à 350 nn est proportionnelle à la quantité d'antigènes effectivement présents dans le dépôt, de sorte que cette fluorescence à 350 nn est une mesure dépendant de la quantité d'antigènes présente dans le dépôt. La quantité de fluorescence à 350 nn est utilisée par le logiciel
   . d'une part pour repérer la position topographique des dépôts d'antigènes sur la lame et
   . d'autre part pour en quantifier la surface exacte et donc les contours afin de ne pas incorporer des artéfacts de fluorescence qui seraient situés en dehors de ces spots, et
   . enfin, pour pondérer la fluorescence à 488 nn. Cette deuxième longueur d'onde de 488 nn, résulte de l'excitation du marqueur de l'Immunoglobuline G de détection. La quantité de fluorescence à 488 nn est en relation avec la quantité d'Immunoglobuline G spécifique présente dans le spot du sérum du patient à tester. La fluorescence à 488 nn (et donc la fixation des Immunoglobulines G) dépend de la quantité d'antigènes qui ont été déposés. En effet, si l'on dépose très peu d'antigène, la quantité d'Immunoglobuline spécifique fixée va être faible et donc le niveau de fluorescence à 488 va être faible. C'est pourquoi la quantité de fluorescence à 488 nn est pondérée pour chacun des spots d'antigènes vaccinaux par la quantité de fluorescence à 350 nn et le ratio des fluorescences exprimant la quantité d'IgG spécifiques présente dans le sérum testé.
8- dans une dernière étape, le logiciel interprète par comparaison des ratios de fluorescence 488/350 mesurés avec les sérums testés avec ceux obtenus avec une collection de sérums de références positifs et négatifs pour chacun qui ont permis d'établir les courbes de référence, l'ensemble de ces données pour indiquer la liste des antigènes vaccinaux contre lesquels le sérum testé possède des anticorps spécifiques, et donc le statut vaccinal de la personne.

Ainsi, dans cet exemple on a testé quatre sérums prélevés chez quatre patients différents pour rechercher la présence d'anticorps de classe IgG contre les antigènes vaccinaux de la rougeole, de la rubéole, des oreillons, de la diphtérie et du tétanos. La valeur seuil du ratio de fluorescence a été déterminée pour chacun de ces 5 antigènes vaccinaux.

Les Tableaux 1A, 1B, 1C et 1D montrent les ratios de fluorescence pour chacun des 4 sérums testés, respectivement.

La lecture des tableaux 1A à 1C indique que :
- le sérum n°1 est :
   - positif pour la présence d'anticorps anti-anatoxine diphtérique (ratio de fluorescence > 1, valeur seuil),
   - positif pour la présence d'anticorps anti-anatoxine tétanique (ratio de fluorescence > 0,05, valeur seuil),
   - négatif pour la présence d'anticorps anti-rubéole (ratio de fluorescence < 0,1, valeur seuil),
   - positif pour la présence d'anticorps anti-rougeole (ratio de fluorescence > 0,1, valeur seuil), et
   - positif pour la présence d'anticorps anti-oreillons (ratio de fluorescence > 0,11, valeur seuil) ;
- le sérum n°2 est :
   - positif pour la présence d'anticorps anti-anatoxine diphtérique (ratio de fluorescence > 1, valeur seuil),
   - positif pour la présence d'anticorps anti-anatoxine tétanique (ratio de fluorescence > 0,05, valeur seuil),
      positif pour la présence d'anticorps anti-rubéole (ratio de fluorescence > 0,1, valeur seuil),
   - positif pour la présence d'anticorps anti-rougeole (ratio de fluorescence > 0,1, valeur seuil), et
   - positif pour la présence d'anticorps anti-oreillons (ratio de fluorescence > 0,11, valeur seuil) ;
- le sérum n°3 est :
   - négatif pour la présence d'anticorps anti-anatoxine diphtérique (ratio de fluorescence < 1, valeur seuil),
   - négatif pour la présence d'anticorps anti-anatoxine tétanique (ratio de fluorescence < 0,05, valeur seuil),
   - négatif pour la présence d'anticorps anti-rubéole (ratio de fluorescence < 0,1, valeur seuil),
   - positif pour la présence d'anticorps anti-rougeole (ratio de fluorescence > 0,1, valeur seuil), et
   - négatif pour la présence d'anticorps anti-oreillons (ratio de fluorescence < 0,11, valeur seuil);
- le sérum n°4 est :
   - négatif pour la présence d'anticorps anti-anatoxine diphtérique (ratio de fluorescence < 1, valeur seuil),
   - négatif pour la présence d'anticorps anti-anatoxine tétanique (ratio de fluorescence < 0,05, valeur seuil),
   - positif pour la présence d'anticorps anti-rubéole (ratio de fluorescence > 0,1, valeur seuil),
   - négatif pour la présence d'anticorps anti-rougeole (ratio de fluorescence < 0,1, valeur seuil), et
   - négatif pour la présence d'anticorps anti-oreillons (ratio de fluorescence égale à 0,11, valeur seuil).

- La figure 1 est un schéma indiquant le plan des dépôts d'antigènes sur la lame vaccin.
- La figure 2 est l'image de cette lame après incubation avec les 4 sérums cités ci-dessus, obtenue à 350 nm (coloration fluorescente non spécifique après marquage fluorescent non-spécifique des protéines à l'AMCA et de l'ADN au colorant Hoescht 332-42). Cette image permet de contrôler la présence de chacun des dépôts d'antigène de contrôle et vaccinaux.

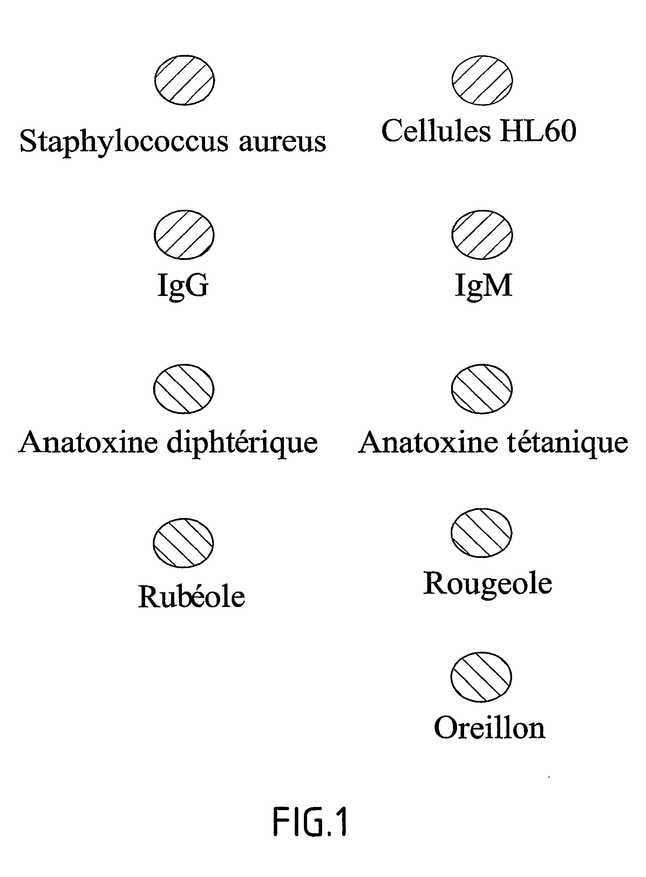

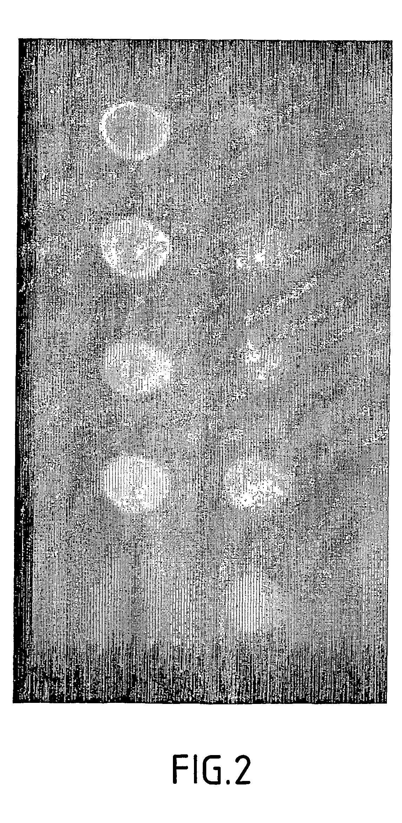

Sur les figures 1 et 2, apparaît un spot (zone de dépôt) IgM qui n'est pas utile dans les tests réalisés.

**Tableau 1A**

| Sérum N° 1 | | | | | | |
|---|---|---|---|---|---|---|
| Numéro | Abscisse | Ordonnée | Surface | Fluorescence 350 | Ratio F350/F488 | Fluorescence 488 |
| SA | 240 | 127 | 1228 | 44238 | 1,274 | 56359 |
| IgG | 238 | 195 | 1198 | 54695 | 0,902 | 49335 |
| Diphtérie | 238 | 263 | 1203 | 44069 | 1,691 | 74521 |
| Rubéole | 235 | 332 | 1117 | 56298 | 0,075 | 4222 |
| HL | 309 | 123 | 452 | 9652 | 0,458 | 4421 |
| IgM | 306 | 197 | 918 | 18369 | 0,183 | 3362 |
| Tétanos | 304 | 266 | 842 | 26512 | 0,078 | 2068 |
| Rougeole | 303 | 334 | 1104 | 37075 | 0,13 | 4820 |
| Oreillon | 302 | 401 | 1038 | 29978 | 0,118 | 3537 |

**Tableau 1B**

| Sérum N° 2 | | | | | | |
|---|---|---|---|---|---|---|
| Numéro | Abscisse | Ordonnée | Surface | Fluorescence 350 | Ratio F488/ F350 | Fluorescence 488 |
| SA | 249 | 109 | 1199 | 41653 | 1,839 | 76600 |
| IgG | 250 | 177 | 1255 | 49420 | 1,263 | 62417 |
| Diphtérie | 251 | 247 | 1176 | 39874 | 2,350 | 93704 |
| Rubéole | 251 | 313 | 1173 | 50395 | 0,108 | 5443 |
| HL | 316 | 106 | 537 | 10853 | 0,627 | 6805 |
| IgM | 318 | 176 | 895 | 15917 | 0,249 | 3963 |
| Tétanos | 321 | 244 | 834 | 21013 | 0,357 | 7502 |
| Rougeole | 319 | 314 | 1164 | 39486 | 0,118 | 4659 |
| Oreillon | 320 | 382 | 1079 | 31342 | 0,120 | 3761 |

**Tableau 1C**

| Sérum N° 3 | | | | | | |
|---|---|---|---|---|---|---|
| Numéro | Abscisse | Ordonnée | Surface | Fluorescence 350 | Ratio F488/F350 | Fluorescence 488 |
| SA | 210 | 85 | 1175 | 34462 | 1,981 | 68269 |
| IgG | 210 | 153 | 1293 | 47678 | 1,561 | 74425 |
| Diphtérie | 210 | 220 | 1255 | 54523 | 0,342 | 18647 |
| Rubéole | 209 | 288 | 1172 | 49101 | 0,073 | 3584 |
| HL | 278 | 78 | 474 | 9180 | 0,740 | 6793 |
| IgM | 278 | 151 | 943 | 15225 | 0,335 | 5100 |
| Tétanos | 278 | 220 | 848 | 24553 | 0,046 | 1129 |
| Rougeole | 278 | 288 | 1178 | 34879 | 0,153 | 5336 |
| Oreillon | 278 | 357 | 1123 | 26949 | 0,095 | 2560 |

**Tableau 1D**

| Sérum N° 4 | | | | | | |
|---|---|---|---|---|---|---|
| Numéro | Abscisse | Ordonnée | Surface | Fluorescence 350 | Ratio F488/F350 | Fluorescence 488 |
| SA | 240 | 110 | 1202 | 40889 | 1,093 | 44692 |
| IgG | 240 | 177 | 1269 | 53557 | 1,123 | 60145 |
| Diphtérie | 242 | 246 | 1253 | 56284 | 0,316 | 17786 |
| Rubéole | 241 | 314 | 1106 | 43970 | 0,107 | 4705 |
| HL | 308 | 106 | 533 | 9932 | 0,797 | 7916 |
| IgM | 308 | 178 | 926 | 17540 | 0,309 | 5420 |
| Tétanos | 309 | 244 | 838 | 29892 | 0,049 | 1465 |
| Rougeole | 309 | 314 | 1174 | 41689 | 0,093 | 3877 |
| Oreillon | 310 | 382 | 1044 | 29666 | 0,110 | 3263 |

## Revendications

1. Méthode de détermination sérologique du statut vaccinal d'un individu par détection et quantification des anticorps sériques du type IgG spécifiques d'antigènes vaccinaux d'une pluralité d'agents pathogènes du type bactéries, virus, champignons ou parasites, **caractérisée en ce que** l'on réalise la détection et la quantification, d'un complexe de réactions immunologiques entre chaque dit antigène vaccinal et respectivement chaque dit anticorps spécifique de type IgG dudit antigène vaccinal, éventuellement présents dans un échantillon de sérum humain à tester, en réalisant les étapes de :
1- mise en contact d'un seul et même dit échantillon de sérum à tester avec :
- un même support solide sur lequel est fixé une pluralité de dits antigènes vaccinaux correspondant à une pluralité d'agents pathogènes différents, de préférence au moins 3, de préférence encore au moins 4 dits antigènes vaccinaux d'agents pathogènes différents, dans des zones différentes du support,
- en présence d'au moins une substance de détection réagissant par complexation avec lesdits anticorps spécifiques de type IgG et ne réagissant pas avec lesdits antigènes vaccinaux, et
2- on détermine la concentration en dits anticorps spécifiques de type IgG par quantification des complexes résultant de la réaction d'au moins une dite substance de détection avec desdits anticorps spécifiques de type IgG complexés auxdits antigènes vaccinaux fixés sur ledit support solide.

2. Méthode selon la revendication 1, **caractérisée en ce que** ladite substance de détection comprend un marquage fluorescent.

3. Méthode selon la revendication 2, **caractérisée en ce que** l'on détermine la concentration de dites immunoglobulines de l'espèce du patient de classe G spécifique dudit antigène vaccinal dans l'échantillon à tester, par lecture automatisée de l'intensité du signal fluorescent émis par ledit élément de marquage fluorescent à l'aide d'un appareil de lecture approprié capable de le quantifier.

4. Méthode selon l'une des revendications 1 à 3, **caractérisée en ce que** lesdits antigènes vaccinaux comprennent au moins 3, de préférence de 3 à 12 antigènes vaccinaux d'agents pathogènes différents choisis parmi les virus des oreillons, de la rubéole, de la rougeole, de la varicelle, de la poliomyélite, de la fièvre jaune, de l'encéphalite à tique, de l'hépatite A, de l'hépatite B, et les bactéries de la coqueluche *Bordetella pertussis,* du tétanos et de la diphtérie.

5. Méthode selon une des revendications 1 à 4, **caractérisée en ce qu'**on détermine si la concentration de dits anticorps spécifiques de type IgG atteint un seuil donné à partir duquel ledit anticorps spécifique a une action protectrice protégeant contre la maladie déterminée par le pathogène.

6. Méthode selon l'une des revendications 1 à 5, **caractérisée en ce qu'**on utilise une même dite substance de détection pour la détection des différents anticorps spécifiques des différents antigènes vaccinaux.

7. Méthode selon la revendication 6, **caractérisée en ce qu'**on met en oeuvre une dite substance de détection qui est une immunoglobuline anti-IgG, de préférence une immunoglobuline de chèvre ou de poussin.

8. Méthode selon l'une des revendications 1 à 5, **caractérisée en ce que** l'on contrôle la présence et la réactivité de ladite première substance de détection en réalisant les étapes de :
- mise en contact dudit échantillon à tester avec un même dit support solide, sur lequel a été fixé en outre un premier antigène de contrôle qui est une immunoglobuline non spécifique de classe G, en présence une solution contenant une dite substance de détection, et
- vérification si ladite substance de détection a réagi avec ledit premier antigène de contrôle fixé sur ledit support solide.

9. Méthode selon l'une des revendications 1 à 8, **caractérisée en ce que** l'on contrôle la présence éventuelle d'un anticorps anti-nucléaire dans ledit échantillon à tester, en
- mettant en contact ledit échantillon à tester avec
• un même dit support solide sur lequel a été fixé un deuxième antigène de contrôle qui comprend des complexes ADN/histones, de préférence comprenant des noyaux de cellules nucléées de cellules de l'espèce du patient, de préférence encore toute ou partie de cellules d'origine de l'espèce du patient en lignée continue, et
• en présence d'une dite substance de détection constituée par un anticorps de marquage qui ne réagit qu'avec une dite immunoglobuline de l'espèce du patient de classe G; et
- en vérifiant si ledit deuxième antigène de contrôle fixé sur le support solide réagit avec ladite substance de détection.

10. Méthode selon l'une des revendications 1 à 9, **caractérisée en ce qu'**on contrôle que ledit échantillon testé contient bien un sérum de l'espèce du patient, en détectant si des immunoglobulines de l'espèce du patient réagissent avec un troisième antigène de contrôle contenant la protéine A d'une bactérie *Staphylococcus aureus,* de préférence en mettant en contact ledit échantillon avec un même dit support solide sur lequel est fixé un dit troisième antigène de contrôle, en présence d'une même dite substance de détection qui est une substance réagissant avec une immunoglobuline de l'espèce du patient et pas avec ledit troisième antigène de contrôle, de préférence un anticorps anti-immunoglobuline de l'espèce du patient et ne réagissant pas avec la protéine A.

11. Méthode selon la revendication 10, **caractérisée en ce que** ledit troisième antigène de contrôle est une bactérie Staphylococcus entière comprenant la protéine A.

12. Méthode selon l'une des revendications 10 ou 11, **caractérisée en ce que** ladite substance de détection est une immunoglobuline de chèvre ou de poussin anti IgG, conjuguée à une substance fluorescente.

13. Méthode selon l'une des revendications 1 à 12, **caractérisée en ce qu'**au moins un dit antigène vaccinal est un antigène microbien corpusculaire constitué de microbe entier inactivé ou fraction de microbe, de préférence fixé sur le support solide par simple dépose et adsorption physique ou liaison physico-chimique avec le support, de préférence en mélange avec un liant protéique.

14. Méthode selon la revendication 13, **caractérisée en ce que** lesdits antigènes corpusculaires vaccinaux et/ou de contrôle sont associés à un colorant, de préférence, un colorant fluorescent.

15. Méthode selon la revendication 13 ou 14, **caractérisée en ce que** ledit liant protéique est choisi parmi du jaune d'oeuf, de la gélatine, de l'albumine de sérum de bovin ou une IgG polyclonale non humaine, de préférence de chèvre.

16. Méthode selon l'une des revendications 13 à 15, **caractérisée en ce que** ledit antigène vaccinal corpusculaire est en mélange avec une immunoglobuline de IgG polyclonale de chèvre.

17. Méthode selon l'une des revendications 1 à 16, **caractérisée en ce qu'**on utilise comme support solide, une lame de verre ou en plastique, ou un puit à fond plat d'une plaque de microtitration en verre ou en plastique.

18. Méthode selon l'une des revendications 1 à 17, **caractérisée en ce que** l'on recueille un volume déterminé de sang total à l'aide d'un tube capillaire dans un flacon contenant un volume déterminé d'un tampon permettant l'élution du sérum, le sérum étant alors de préférence dilué à une concentration déterminée, de préférence de 1/100 à 1/20, et l'on dépose un volume déterminé de sérum ainsi dilué sur les différentes zones de dépôt desdits antigènes de contrôle et antigènes vaccinaux sur ledit support solide.

19. Méthode selon l'une des revendications 1 à 18, **caractérisée en ce que**, pour chaque détection et, le cas échéant, quantification d'un dit antigène vaccinal, on réalise les mesures suivantes :
1- une première mesure d'une première valeur représentative de la quantité d'un premier élément de marquage de préférence la première valeur de l'intensité d'un signal émis par ledit premier élément de marquage fluorescent, ledit premier élément de marquage se fixant de manière non spécifique sur toute protéine dans la zone de dépôt dudit antigène vaccinal, et
2- une deuxième mesure d'une deuxième valeur représentative de la quantité d'un deuxième élément de marquage différent dudit premier élément de marquage et émettant un signal différent, de préférence une deuxième valeur de l'intensité du signal émis par ce deuxième élément de marquage fluorescent à une longueur d'onde d'excitation différente de celle dudit premier élément de marquage fluorescent, ledit deuxième élément de marquage étant l'élément de marquage de ladite substance de détection dudit antigène vaccinal, dans la zone de dépôt dudit antigène, et
3- on calcule le rapport desdites première et deuxième valeurs, et
4- on compare la valeur dudit rapport à celle d'un rapport de référence obtenu avec une collection de sérums de référence positifs et négatifs, permettant ainsi par comparaison de déterminer la nécessité ou non de vacciner la personne pour ledit antigène vaccinal selon la valeur du rapport desdites première et deuxième valeurs.

## Claims

1. Serological method for determining the vaccine status of a person by detection and quantification of IgG-type serum antibodies specific to the vaccine antigens of a plurality of pathogenic agents of bacterial, viral, fungal or parasitic type, **characterized in that** a complex of immunological reactions is detected and quantified between each said vaccine antigen and respectively each said IgG-type antibody specific to said vaccine antigen, possibly present in a sample of human serum to be tested, by conducting the steps of:
1-Contacting one same said serum sample to be tested with:
- one same solid substrate on which a plurality of said vaccine antigens are fixed corresponding to a plurality of different pathogenic agents, preferably at least 3, further preferably at least 4 said vaccine antigens of different pathogenic agents, at different areas of the substrate,
- in the presence of at least one detection substance reacting by complexing with said IgG-type specific antibodies and not reacting with said vaccine antigens, and
2- The concentration of said IgG-type specific antibodies is determined by quantifying the complexes resulting from the reaction of at least one said detection substance with said IgG-type specific antibodies complexed with said vaccine antigens fixed onto said solid substrate.

2. Method according to claim 1, **characterized in that** said detection substance contains fluorescent labelling.

3. Method according to claim 2, **characterized in that** the concentration of said class G immunoglobulins of the patient species specific to said vaccine antigen is determined in the sample to be tested, by automated reading of the intensity of the fluorescent signal emitted by said fluorescent labelling element using appropriate reading apparatus able to quantify the same.

4. Method according to any of claims 1 to 3, **characterized in that** said vaccine antigens comprise at least 3, preferably 3 to 12 vaccine antigens of different pathogenic agents chosen from among the following viruses: mumps, rubella, measles, chicken pox, poliomyelitis, yellow fever, tick-borne encephalitis, hepatitis A, hepatitis B, and from the following bacteria: *Bordetella pertussis,* tetanus and diphtheria.

5. Method according to any of claims 1 to 4, **characterized in that** it is determined whether the concentration of said IgG-type specific antibodies reaches a given threshold on and after which said specific antibody has a protective action protecting against the disease determined by the pathogen.

6. Method according to any of claims 1 to 5, **characterized in that** one same detection substance is used to detect the different specific antibodies of the different vaccine antigens.

7. Method according to claim 6, **characterized in that** said detection substance used is an anti-IgG immunoglobulin, preferably a goat or chick immunoglobulin.

8. Method according to any of claims 1 to 5, **characterized in that** the presence and reactivity of said detection substance is controlled by conducting the steps of:
- contacting said sample to be tested with one same said solid substrate, on which a first control antigen has also been fixed which is a class G non-specific immunoglobulin, in the presence of a solution containing a said detection substance, and
- verifying whether said detection substance has reacted with said first control antigen fixed on said solid substrate.

9. Method according to any of claims 1 to 8, **characterized in that** the possible presence of an antinuclear antibody is controlled in said sample to be tested, by:
• contacting said sample to be tested with:
- one same said solid substrate on which a second control antigen has been fixed which comprises DNA/histone complexes, preferably comprising nuclei of nucleated cells of patient species cells, further preferably all or part of continuous line patient species cells, and
- in the presence of a said detection substance consisting of a labelling antibody which only reacts with said class G immunoglobulin of the patient species; and
• verifying whether said control antigen fixed on the solid substrate reacts with said detection substance.

10. Method according to any of claims 1 to 9, **characterized in that** it is controlled that said tested sample effectively contains a serum of the patient species, by detecting whether immunoglobulins of the patient species react with a third control antigen containing protein A of a *Staphylococcus aureus* bacterium, preferably by contacting said sample with the same said solid substrate on which a said third control antigen is fixed, in the presence of a same said detection substance which is a substance reacting with an immunoglobulin of the patient species and not with said third control antigen, preferably an anti-immunoglobulin antibody of the patient species, and not reacting with protein A.

11. Method according to claim 10, **characterized in that** said third control antigen is a whole *Staphylococcus* bacterium containing protein A.

12. Method according to either of claims 10 or 11, **characterized in that** said detection substance is an anti-IgG goat or chick immunoglobulin, conjugated with a fluorescent substance.

13. Method according to any of claims 1 to 12, **characterized in that** at least one said vaccine antigen is a corpuscular microbial antigen consisting of a whole inactivated microbe or microbe fraction, preferably fixed onto the solid substrate by simple deposit and physical adsorption or physicochemical binding with the substrate, preferably in a mixture with a protein binder.

14. Method according to claim 13, **characterized in that** said corpuscular vaccine and/or control antigens are associated with a dye, preferably a fluorescent dye.

15. Method according to claim 13 or 14, **characterized in that** said protein binder is chosen from among egg yolk, gelatine, bovine serum albumin or a non-human polyclonal IgG, preferably goat.

16. Method according to any of claims 13 to 15, **characterized in that** said corpuscular vaccine antigen is in a mixture with a goat polyclonal IgG-type immunoglobulin.

17. Method according to any of claims 1 to 16, **characterized in that** as solid substrate a glass or plastic slide is used, or a flat-bottomed well of a microtitration plate in glass or plastic.

18. Method according to any of claims 1 to 17, **characterized in that** a determined volume of whole blood is collected using a capillary tube in a bottle containing a determined volume of buffer allowing elution of the serum, the serum then preferably being diluted to a determined concentration, preferably 1/100 to 1/20, and a determined volume of serum so diluted is deposited on the different deposit areas of said control antigens and vaccine antigens on said solid substrate.

19. Method according to any of claims 1 to 18, **characterized in that** for each detection, and optionally each quantification, of a said vaccine antigen the following measurements are made:
1-a first measurement of a first value representing the quantity of a first labelling element, preferably the first value of the intensity of a signal emitted by said first fluorescent labelling element, said first labelling element fixing non-specifically to any protein in the deposit area of said vaccine antigen, and
2- a second measurement of a second value representing the quantity of a second labelling element, different from said first labelling element and emitting a different signal, preferably a second value of the intensity of the signal emitted by this second fluorescent labelling element at a different excitation wavelength to that of said first fluorescent labelling element, said second labelling element being the labelling element of said detection substance of said vaccine antigen, in the deposit area of said antigen, and
3- the ratio of said first and second values is calculated, and
4- the value of said ratio is compared with value of a reference ratio obtained from a collection of positive and negative reference sera, thereby making it possible to determine, by comparison, whether or not there is a need to vaccinate the person for said vaccine antigen according to the ratio of said first and second values.

## Patentansprüche

1. Serologisches Verfahren zum Bestimmen des Impfstatus eines Individuums durch Nachweis und Quantifizierung von Serumantikörpern vom Typ IgG, die für Impfstoffantigene einer Vielzahl von Erregern vom Typ Bakterien, Viren, Pilze oder Parasiten spezifisch sind, **dadurch gekennzeichnet, daß** der Nachweis und die Quantifizierung eines Komplexes immunologischer Reaktionen zwischen jedem der Impfstoffantigene bzw. jedem der spezifischen Antikörper vom Typ IgG des Impfstoffantigens ausgeführt wird, die gegebenenfalls in einer zu testenden Humanserumprobe vorhanden sind, indem die folgenden Schritte ausgeführt werden:
1- In-Kontakt-Bringen ein und derselben zu testenden Serumprobe mit:
- einem gleichen festen Träger, auf dem eine Vielzahl von Impfstoffantigenen fixiert ist, die einer Vielzahl von Erregern entsprechen, bevorzugt mindestens 3, bevorzugter mindestens 4 Impfstoffantigene verschiedener Erreger, auf verschiedenen Zonen des Trägers,
- in Gegenwart mindestens einer Nachweissubstanz, die durch Komplexbildung mit den spezifischen Antikörpern vom Typ IgG reagiert und mit den Impfstoffantigenen nicht reagiert, und
2- Bestimmen der Konzentration an spezifischen Antikörpern vom Typ IgG durch Quantifizieren der Komplexe, die aus der Reaktion mindestens einer der Nachweissubstanzen mit den spezifischen Antikörpern vom Typ IgG resultieren, die einen Komplex mit den Impfstoffantigenen bilden, die auf dem festen Träger fixiert sind.

2. Verfahren nach Anspruch 1, **dadurch gekennzeichnet, daß** die Nachweissubstanz eine fluoreszierende Markierung umfaßt.

3. Verfahren nach Anspruch 2, **dadurch gekennzeichnet, daß** die Konzentration der Immunoglobuline der Spezies des Patienten der Klasse G, die für das Impfstoffantigen in der zu testenden Probe spezifisch ist, durch automatisiertes Lesen der Intensität des Fluoreszenzsignals, das von dem Element zur fluoreszierenden Markierung emittiert wird, mit Hilfe einer geeigneten Lesevorrichtung, die in der Lage ist, es zu quantifizieren, bestimmt wird.

4. Verfahren nach einem der Ansprüche 1 bis 3, **dadurch gekennzeichnet, daß** die Impfstoffantigene mindestens 3, bevorzugt 3 bis 12 Impfstoffantigene der verschiedenen Erreger, ausgewählt aus Mumpsvirus, Rötelnvirus, Masernvirus, Windpockenvirus, Poliomyelitisvirus, Gelbfiebervirus, Zeckenenzephalitisvirus, Hepatitis-A-Virus, Hepatitis-B-Virus und Keuchhustenbakterien *Bordetella pertussis,* Tetanusbakterien und Diphteriebakterien, umfassen.

5. Verfahren nach einem der Ansprüche 1 bis 4, **dadurch gekennzeichnet, daß** bestimmt wird, ob die Konzentration der spezifischen Antikörper vom Typ IgG einen gegebenen Schwellenwert erreicht, ab dem der spezifische Antikörper eine schützende Wirkung aufweist, die gegen die durch das Pathogen bestimmte Krankheit schützt.

6. Verfahren nach einem der Ansprüche 1 bis 5, **dadurch gekennzeichnet, daß** eine gleiche Nachweissubstanz zum Nachweis der verschiedenen spezifischen Antikörper der verschiedenen Impfstoffantigene verwendet wird.

7. Verfahren nach Anspruch 6, **dadurch gekennzeichnet, daß** eine Nachweissubstanz eingesetzt wird, die ein Anti-IgG-Immunoglobulin, stärker bevorzugt ein Immunoglobulin von der Ziege oder vom Huhn ist.

8. Verfahren nach einem der Ansprüche 1 bis 5, **dadurch gekennzeichnet, daß** die Gegenwart und die Reaktionsfähigkeit der ersten Nachweissubstanz kontrolliert werden, indem die folgenden Schritte ausgeführt werden:
- In-Kontakt-Bringen der zu testenden Probe mit einem gleichen festen Träger, auf dem ferner ein erstes Kontrollantigen fixiert wurde, das ein unspezifisches Immunoglobulin der Klasse G ist, in Gegenwart einer Lösung, die eine Nachweissubstanz enthält, und
- Überprüfen, ob die erste Nachweissubstanz mit dem ersten Kontrollantigen, das auf dem festen Träger fixiert ist, reagiert hat.

9. Verfahren nach einem der Ansprüche 1 bis 8, **dadurch gekennzeichnet, daß** die mögliche Gegenwart eines antinuklären Antikörpers in der zu testenden Probe kontrolliert wird, indem
- die zu testende Probe in Kontakt gebracht wird mit
• einem gleichen festen Träger, auf dem ein zweites Kontrollantigen fixiert wurde, das DNA-/Histon-Komplexe umfaßt, die bevorzugt Nuclei von nucleierten Zellen der Spezies des Patienten, stärker bevorzugt die vollständigen Zellen oder einen Teil der Zellen umfassen, die von der Spezies des Patienten in kontinuierlicher Linie stammen, und
• in Gegenwart einer Nachweissubstanz, die aus einem Markierungsantikörper besteht, der nur mit einem Immunoglobulin der Spezies des Patienten der Klasse G reagiert; und
- indem überprüft wird, ob das zweite Kontrollantigen, das auf dem festen Träger fixiert ist, mit der Nachweissubstanz reagiert.

10. Verfahren nach einem der Ansprüche 1 bis 9, **dadurch gekennzeichnet, daß** kontrolliert wird, ob die getestete Probe tatsächlich ein Serum der Spezies des Patienten enthält, indem nachgewiesen wird, ob Immunoglobuline der Spezies des Patienten mit einem dritten Kontrollantigen reagieren, das das Protein A eines *Staphylococcus-aureus*-Bakteriums, enthält, bevorzugt indem die Probe mit einem gleichen festen Träger in Kontakt gebracht wird, auf dem ein drittes Kontrollantigen fixiert ist, in Gegenwart einer gleichen Nachweissubstanz, die eine Substanz ist, die mit einem Immunoglobulin der Spezies des Patienten und nicht mit dem dritten Kontrollantigen reagiert, die bevorzugt mit einem Anti-Immunoglobulin-Antikörper der Spezies des Patienten und nicht mit dem Protein A reagiert.

11. Verfahren nach Anspruch 10, **dadurch gekennzeichnet, daß** das dritte Kontrollantigen eine vollständige Staphylococcus-Bakterie ist, die das Protein A umfaßt.

12. Verfahren nach einem der Ansprüche 10 oder 11, **dadurch gekennzeichnet, daß** die Nachweissubstanz ein Anti-IgG-Immunoglobulin von der Ziege oder vom Huhn ist, das an eine fluoreszierende Substanz konjugiert ist.

13. Verfahren nach einem der Ansprüche 1 bis 12, **dadurch gekennzeichnet, daß** mindestens ein Impfstoffantigen ein korpuskuläres mikrobielles Antigen ist, das aus einer inaktivierten vollständigen Mikrobe oder einer Mikrobenfraktion besteht, das bevorzugt auf dem festen Träger durch einfaches Auftragen und physikalische Adsorption oder physikalisch-chemische Verbindung mit dem Träger, bevorzugt als Gemisch mit einem Proteinbindemittel, fixiert ist.

14. Verfahren nach Anspruch 13, **dadurch gekennzeichnet, daß** die korpuskulären Impfstoff- oder Kontrollantigene mit einem Farbstoff, bevorzugt einem Fluoreszenzfarbstoff, assoziiert sind.

15. Verfahren nach Anspruch 13 oder 14, **dadurch gekennzeichnet, daß** das Proteinbindemittel aus Eigelb, Gelatine, Rinderserumalbumin oder einem polyklonalen nicht humanen IgG, bevorzugt von der Ziege, ausgewählt ist.

16. Verfahren nach einem der Ansprüche 13 bis 15, **dadurch gekennzeichnet, daß** das korpuskuläre Impfstoffantigen als Gemisch mit einem polyklonalen IgG-Immunoglobulin von der Ziege vorliegt.

17. Verfahren nach einem der Ansprüche 1 bis 16, **dadurch gekennzeichnet, daß** als fester Träger ein Objektträger aus Glas oder Kunststoff oder eine Vertiefung mit flachem Boden einer Mikrotiterplatte aus Glas oder Kunststoff verwendet wird.

18. Verfahren nach einem der Ansprüche 1 bis 17, **dadurch gekennzeichnet, daß** ein bestimmtes Gesamtblutvolumen mit Hilfe einer Kapillarröhre in einem Kolben gesammelt wird, der ein bestimmtes Volumen eines Puffers enthält, der das Eluieren des Serums ermöglicht, wobei das Serum dann auf eine bestimmte Konzentration, bevorzugt zwischen 1/100 und 1/20 verdünnt wird, und ein bestimmtes Volumen des so verdünnten Serums auf die verschiedenen Auftragszonen der Kontrollantigene und Impfstoffantigene auf dem festen Träger aufgetragen werden.

19. Verfahren nach einem der Ansprüche 1 bis 18, **dadurch gekennzeichnet, daß** für jeden Nachweis und gegebenenfalls jede Quantifizierung eines Impfstoffantigens die folgenden Messungen ausgeführt werden:
1- eine erste Messung eines ersten Werts, der für die Menge eines ersten Markierungselement repräsentativ ist, bevorzugt für den ersten Wert der Intensität eines Signals, das von dem ersten fluoreszierenden Markierungselement emittiert wird, wobei sich das erste Markierungselement unspezifisch an jedes Protein in der Auftragszone des Impfstoffantigens anheftet, und
2- eine zweite Messung eines zweiten Werts, der für die Menge eines zweiten Markierungselements repräsentativ ist, das vom ersten Markierungselement verschieden ist und ein verschiedenes Signal emittiert, bevorzugt einen zweiten Wert der Intensität des Signals, das von diesem zweiten fluoreszierenden Markierungselement emittiert wird, mit einer Anregungswellenlänge, die von derjenigen des ersten fluoreszierenden Markierungselements verschieden ist, wobei das zweite Markierungselement das Markierungselement der Nachweissubstanz des Impfstoffantigens in der Auftragszone des Antigens ist, und
3- Berechnen des Verhältnisses zwischen dem ersten und dem zweiten Wert, und
4- Vergleichen des Werts des Verhältnisses mit dem Wert eines Referenzverhältnisses, das bei einer Sammlung positiver und negativer Referenzseren erhalten wurde, wodurch es durch Vergleich möglich ist, je nach dem Wert des Verhältnisses zwischen dem ersten und dem zweiten Wert, zu bestimmen, ob es notwendig ist oder nicht, die Person für das Impfstoffantigen zu impfen.
